(19) Europäisches Patentamt
European Patent Office
Office européen des brevets

(11) Veröffentlichungsnummer: **0 374 757**
**A1**

# (12) EUROPÄISCHE PATENTANMELDUNG

(21) Anmeldenummer: **89123244.9**

(22) Anmeldetag: **15.12.89**

(51) Int. Cl.5: **C07D 233/22, A01N 43/50, C07D 233/20, C07D 233/24**

Patentansprüche für folgenden Vertragsstaat: ES.

(30) Priorität: **20.12.88 DE 3842798**

(43) Veröffentlichungstag der Anmeldung: **27.06.90 Patentblatt 90/26**

(84) Benannte Vertragsstaaten: **AT BE CH DE ES FR GB GR IT LI NL SE**

(71) Anmelder: **HOECHST AKTIENGESELLSCHAFT**
**Postfach 80 03 20**
**D-6230 Frankfurt am Main 80(DE)**

(72) Erfinder: **Stark, Herbert, Dr.**
**Gimbacher Tann, 15**
**D-6233 Kelkheim Taunus(DE)**
Erfinder: **Salbeck, Gerhard, Dr.**
**Königsberger Strasse 52**
**D-6239 Kriftel(DE)**
Erfinder: **Bonin, Werner, Dr.**
**Im Schulzehnten 18**
**D-6233 Kelkheim Taunus(DE)**

## (54) Imidazolinderivate enthaltende Mittel zur wirtstiersystemischen Bekämpfung von Ektoparasiten sowie neue Imidazolinderivate.

(57) Gegenstand der vorliegenden Erfindung sind Mittel zur wirtstiersystemischen Bekämpfung von Ektoparasiten, dadurch gekennzeichnet, daß sie eine Verbindung der Formel I

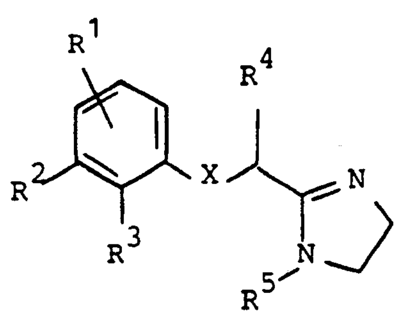

(I),

worin
$R^1$ Wasserstoff, ($C_1$-$C_5$)-Alkyl, ($C_1$-$C_3$)-Halogenalkyl oder Halogen;
$R^2$, $R^3$ unabhängig voneinander ($C_1$-$C_5$)-Alkyl, ($C_2$-$C_5$)Alkenyl, ($C_2$-$C_5$)-Alkinyl, ($C_3$-$C_7$)-Cycloalkyl, ($C_3$-$C_7$)-Cycloalkenyl, ($C_1$-$C_3$)-Halogenalkyl, Halogen Cyano, Nitro, ($C_1$-$C_5$)-Alkoxy, ($C_1$-$C_3$)-Alkoxy-($C_1$-$C_3$)-alkyl, ($C_1$-$C_3$)-Halogenalkoxy, ($C_1$-$C_3$)-Alkylthio,
oder $R^2$ und $R^3$ bilden zusammen eine Polymethylenkette mit 2 bis 5 C-Atomen;
$R^4$ Wasserstoff, ($C_1$-$C_{10}$)-Alkyl, ($C_2$-$C_5$)-Alkenyl, ($C_3$-$C_7$)-Cycloalkyl;
$R^5$ Wasserstoff, ($C_1$-$C_{10}$)-Alkyl, ($C_3$-$C_5$)-Alkenyl, ($C_3$-$C_5$)-Alkinyl, ($C_3$-$C_7$)-Cycloalkyl, ($C_3$-$C_7$)-Cycloalkenyl, ($C_1$-$C_3$)-Halogenalkyl oder ($C_1$-$C_3$)-Alkoxy-($C_1$-$C_3$)-alkyl,
X Sauerstoff, Schwefel oder eine -$NR^6$-Gruppe;
$R^6$ Wasserstoff, ($C_1$-$C_5$)-Alkyl, ($C_3$-$C_5$)-Alkenyl, ($C_3$-$C_5$)-Alkinyl, ($C_3$-$C_7$)-Cycloalkyl, ($C_3$-$C_7$)-Cycloalkenyl, ($C_1$-$C_3$)-Halogenalkyl oder ($C_1$-$C_3$)-Alkoxy-($C_1$-$C_3$)-alkyl
bedeuten, sowie deren biologisch verträgliche Säureadditionssalze enthalten.

EP 0 374 757 A1

Die Verbindungen der Formel I sind zum Teil neu und werden ebenfalls von der Erfindung umfaßt.

# Imidazolinderivate enthaltende Mittel zur wirtstiersystemischen Bekämpfung von Ektoparasiten sowie neue Imidazolinderivate

Imidazolinderivate der nachstehenden Formel I sind teilweise bekannt und ihre insektizide oder acarizide Wirksamkeit vorbeschrieben (AU-PS 506 440, DE-OS 2750902, 2756638 und 2818367).

Die dort beschriebenen Methoden zur Schädlingsbekämpfung beruhen jedoch immer darauf, daß die Schädlinge mit einem den Wirkstoff enthaltenden Mittel besprüht bzw., im Fall von Ektoparasiten, in dieses Mittel eingetaucht werden, d. h. die Schädlinge werden in irgendeiner Form äußerlich mit dem entsprechenden Mittel in Kontakt gebracht. Dies entspricht auch der üblichen Ektoparasitenbekämpfung in der Praxis, bei der die befallenen Wirtstiere besprüht, in Bäder getaucht oder mit pour-on Lösungen behandelt werden.

Es wurde nun überraschenderweise gefunden, daß Verbindungen der Formel I vorteilhaft zur wirtstiersystemischen Bekämpfung von Ektoparasiten geeignet sind. Gegenstand der vorliegenden Erfindung sind daher Mittel zur wirtstiersystemischen Bekämpfung von Ektoparasiten, dadurch gekennzeichnet, daß sie eine Verbindung der Formel I

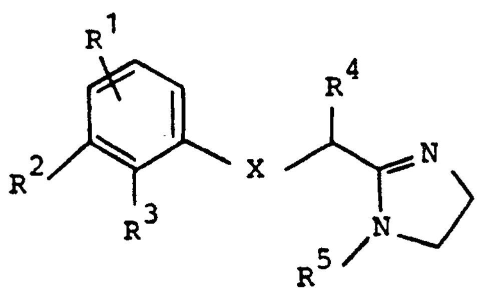

(I),

worin

$R^1$ Wasserstoff, $(C_1-C_5)$-Alkyl, $(C_1-C_3)$-Halogenalkyl oder Halogen;

$R^2$, $R^3$ unabhängig voneinander $(C_1-C_5)$-Alkyl, $(C_2-C_5)$Alkenyl, $(C_2-C_5)$-Alkinyl, $(C_3-C_7)$-Cycloalkyl, $(C_3-C_7)$-Cycloalkenyl, $(C_1-C_3)$-Halogenalkyl, Halogen Cyano, Nitro, $(C_1-C_5)$-Alkoxy, $(C_1-C_3)$-Alkoxy-$(C_1-C_3)$-alkyl, $(C_1-C_3)$-Halogenalkoxy, $(C_1-C_3)$-Alkylthio,

oder $R^2$ und $R^3$ bilden zusammen eine Polymethylenkette mit 2 bis 5 C-Atomen;

$R^4$ Wasserstoff, $(C_1-C_{10})$-Alkyl, $(C_2-C_5)$-Alkenyl, $(C_3-C_7)$-Cycloalkyl;

$R^5$ Wasserstoff, $(C_1-C_{10})$-Alkyl, $(C_3-C_5)$-Alkenyl, $(C_3-C_5)$-Alkinyl, $(C_3-C_7)$-Cycloalkyl, $(C_3-C_7)$-Cycloalkenyl, $(C_1-C_3)$-Halogenalkyl oder $(C_1-C_3)$-Alkoxy-$(C_1-C_3)$-alkyl,

X Sauerstoff, Schwefel oder eine -$NR^6$-Gruppe;

$R^6$ Wasserstoff, $(C_1-C_5)$-Alkyl, $(C_3-C_5)$-Alkenyl, $(C_3-C_5)$-Alkinyl, $(C_3-C_7)$-Cycloalkyl, $(C_3-C_7)$-Cycloalkenyl, $(C_1-C_3)$-Halogenalkyl oder $(C_1-C_3)$-Alkoxy-$(C_1-C_3)$-alkyl

bedeuten, oder deren biologisch verträgliche Säureadditionssalze enthalten.

Dabei bedeutet Alkyl sowohl geradkettiges als auch verzweigtes Alkyl, und Halogen bedeutet bevorzugt F oder Cl. Bevorzugt unter den biologisch verträglichen Säureadditionssalzen der Verbindungen I sind die Hydrohalogenide, insbesondere die Hydrochloride. Die erfindungsgemäßen Mittel können auch mehrere Wirkstoffe I enthalten.

Die erfindungsgemäßen Verbindungen können auch zur simultanen Bekämpfung von Endo- und Ektoparasiten in Kombination mit systemisch wirksamen Präparaten, wie z. B. den Avermectinen oder mit Levamisol oder Netobimin, oder, bei oraler Applikation, in Kombination mit Präparaten aus der Gruppe der Benzimidazole, wie z. B. Phenbendazol oder Albendazol, vorteilhaft verwendet werden.

Bevorzugte Verbindungen der Formel I sind diejenigen, bei denen

$R^1$ Wasserstoff, $(C_1-C_3)$-Alkyl, $(C_1-C_3)$-Halogenalkyl, F oder Cl;

$R^2$, $R^3$ unabhängig voneinander $(C_1-C_3)$-Alkyl, $(C_2-C_3)$-Alkenyl, $(C_2-C_3)$-Alkinyl, $(C_3-C_5)$-Cycloalkyl, $(C_1-C_3)$-Halogenalkyl, Halogen, Cyano, Nitro, $(C_1-C_3)$-Alkoxy, $(C_1-C_3)$-Alkoxy-$(C_1-C_2)$-alkyl, $(C_1-C_3)$-Halogenalkoxy, $(C_1-C_3)$-Alkylthio, oder $R^2$ und $R^3$ bilden zusammen eine Polymethylenkette mit 2 bis 5 C-Atomen;

$R^4$ Wasserstoff, $(C_1-C_4)$-Alkyl, $(C_2-C_3)$-Alkenyl, $(C_3-C_6)$-Cycloalkyl;

$R^5$ Wasserstoff, $(C_1-C_4)$-Alkyl, $(C_3-C_4)$-Alkenyl, $(C_3-C_4)$-Alkinyl, $(C_3-C_6)$-Cycloalkyl, $(C_3-C_5)$-Cycloalkenyl, $(C_1-C_3)$-Halogenalkyl oder $(C_1-C_3)$-Alkoxy-$(C_1-C_2)$-alkyl

X Sauerstoff oder eine -$NR^6$-Gruppe;

$R^6$ Wasserstoff, $(C_1-C_4)$-Alkyl, $(C_3-C_4)$-Alkenyl, $(C_3-C_4)$-Alkinyl, $(C_3-C_6)$-Cycloalkyl, $(C_1-C_3)$-Halogenalkyl

oder ($C_1$-$C_3$)-Alkoxy-($C_1$-$C_2$)-alkyl
bedeuten, sowie deren biologisch verträgliche Säureadditionssalze.

Diese Säureadditionssalze stellen die Hauptanwendungsform der Verbindungen I dar.

Insbesondere sind die nachstehenden Hydrohalogenide Nr. 1 -115 zu erwähnen:

1 2-(2,5-Dichlor-3-trifluormethylphenyl-aminomethyl)-2-imidazolin-hydrochlorid
2 2-(2,6-Dichlor-3-methylphenyl-aminomethyl)-2-imidazolin-hydrochlorid
3 2-(6-Fluor-2,3-dimethylphenyl-aminomethyl)-2-imidazolin-hydrochlorid
4 2-(2-Chlor-4-fluor-3-methylphenyl-aminomethyl)-2-imidazolin-hydrochlorid
5 2-(2-Chlor-4-fluor-3-trifluormethylphenyl-aminomethyl)-2-imidazolin-hydrochlorid
6 2-(2,4-Dichlor-3-fluorphenyl-aminomethyl)-2-imidazolin-hydrochlorid
7 2-(4-Methoxy-2,3-dimethylphenyl-aminomethyl)-2-imidazolin-hydrochlorid
8 2-(2,3,4-Trimethylphenyl-aminomethyl)-2-imidazolin-hydrochlorid
9 1-Ethyl-2-(6-fluor-2,3-dimethylphenyl-aminomethyl)-2-imidazolin-hydrochlorid
10 2-(2-Methyl-3-vinylphenyl-aminomethyl)-2-imidazolin-hydrochlorid
11 2-(2-Methyl-3-allylphenyl-aminomethyl)-2-imidazolin-hydrochlorid
12 1-Methyl-2-(2-allyl-3-methylphenyl-aminomethyl)-2-imidazolin-hydrochlorid
13 2-(3-Ethinyl-2-methylphenyl-aminomethyl)-2-imidazolin-hydrochlorid
14 2-(2-Cyclopropyl-3-methylphenyl-aminomethyl)-2-imidazolin-hydrochlorid
15 2-(2-Chlor-3-cyclopropylphenyl-aminomethyl)-2-imidazolin-hydrochlorid
16 2-(3-Cyclopentyl-2-methylphenyl-aminomethyl)-2-imidazolin-hydrochlorid
17 2-(3-Cyclopent-1-en-2-methylphenyl-aminomethyl)-2-imidazolin-hydrochlorid
18 2-(3-Trifluormethyl-2-methylphenyl-aminomethyl)-2-imidazolin-hydrochlorid
19 2-(3-Nitro-2-methylphenyl-aminomethyl)-2-imidazolin-hydrochlorid
20 2-(3-Nitro-2-chlorphenyl-aminomethyl)-2-imidazolin-hydrochlorid
21 2-(2-Nitro-3-methylphenyl-aminomethyl)-2-imidazolin-hydrochlorid
22 2-(3-Methoxymethyl-2-methylphenyl-aminomethyl)-2-imidazolin-hydrochlorid
23 1-Ethyl-2-(3-methoxymethyl-2-methylphenyl-aminomethyl)-2-imidazolin-hydrochlorid
24 2-(3-Chlor-2-difluormethoxyphenyl-aminomethyl)-2-imidazolin-hydrochlorid
25 2-(3-Difluormethoxy-2-methylphenyl-aminomethyl)-2-imidazolin-hydrochlorid
26 2-(2-Chlor-3-tetrafluorethoxy-phenyl-aminomethyl)-2-imidazolin-hydrochlorid
27 2-(2-Methyl-3-methylthiophenyl-aminomethyl)-2-imidazolin-hydrochlorid
28 2-((1,2,3,4-Tetrahydronaphth-5-yl)-aminomethyl)-2-imidazolin-hydrochlorid
29 2-(Indan-4-yl-aminomethyl)-2-imidazolin-hydro chlorid
30 2-(3-Allyl-2-methylphenoxy-methyl)-2-imidazolin-hydrochlorid
31 2-(3-Cyclopropyl-2-methylphenoxy-methyl)-2-imidazolin-hydrochlorid
32 2-(3-Trifluormethyl-2-methylphenoxy-methyl)-2-imidazolin-hydrochlorid
33 2-(2-Cyano-3-methylphenoxy-methyl)-2-imidazolin-hydrochlorid
34 2-(3-Methoxymethyl-2-methylphenoxy-methyl)-2-imidazolin-hydrochlorid
35 2-(3-Difluormethoxy-2-methylphenoxy-methyl)-2-imidazolin-hydrochlorid
36 2-(2-Ethyl-3-methylphenyl-aminomethyl)-2-imidazolin-hydrochlorid
37 2-(2,3-Diethylphenyl-aminomethyl)-2-imidazolin-hydrochlorid
38 2-(3-Ethyl-2-methylphenyl-aminomethyl)-2-imidazolin-hydrochlorid
39 2-(3-Fluor-2-methylphenyl-aminomethyl)-2-imidazolin-hydrochlorid
40 2-(3-Brom-2-methylphenyl-aminomethyl)-2-imidazolin-hydrochlorid
41 2-(3-Chlor-2-fluorphenyl-aminomethyl)-2-imidazolin-hydrochlorid
42 2-(3-Nitro-2-methylphenoxy-methyl)-2-imidazolin-hydrochlorid
43 2-(3-Ethyl-2-methylphenoxy-methyl)-2-imidazolin-hydrochlorid
44 2-(3-Fluor-2-methylphenoxy-methyl)-2-imidazolin-hydrochlorid
45 2-(3-Brom-2-methylphenoxy-methyl)-2-imidazolin-hydrochlorid
46 2-(3-Chlor-2-fluorphenoxy-methyl)-2-imidazolin-hydrochlorid
47 2-(1-(2,3-Dimethylphenyl-amino)-prop-2-enyl)-2-imidazolin-hydrochlorid
48 2-(Cyclopropyl-(2,3-dimethylphenyl-amino)-methyl)-2-imidazolin-hydrochlorid
49 2-(1-(2,3-Dimethylphenyl-amino)-ethyl)-2-imidazolin-hydrochlorid
50 2-(1-(2,3-Dimethylphenyl-N-methyl-amino)-ethyl)-2-imidazolin-hydrochlorid
51 1-Methyl-2-(1-(2,3-dimethylphenyl-amino)-ethyl)-2-imidazolin-hydrochlorid
52 1-Ethyl-2-(1-(2,3-dimethylphenyl-amino)-ethyl)-2-imidazolin-hydrochlorid
53 1-Methyl-2-(1-(2,3-dimethylphenyl-N-methyl-amino)-ethyl)-2-imidazolin-hydrochlorid
54 1-Ethyl-2-(1-(2,3-dimethylphenyl-N-methyl-amino)-ethyl)-2-imidazolin-hydrochlorid

55 2-(2,3-Dimethylphenyl-thiomethyl)-2-imidazolin-hydrochlorid
56 2-(3-Chlor-2-methylphenyl-thiomethyl)-2-imidazolin-hydrochlorid
57 2-(2-Chlor-3-methylphenyl-thiomethyl)-2-imidazolin-hydrochlorid
58 1-Ethyl-2-(3-chlor-2-methylphenyl-thiomethyl)-2-imidazolin-hydrochlorid
59 2-(1-(2,3-Dimethylphenyl-thio)-ethyl)-2-imidazolin-hydrochlorid
60 1-Ethyl-2-(2,3-dimethylphenyl-aminomethyl)-2-imidazolin-hydrochlorid
61 1-Ethyl-2-(2,3-dimethylphenyl-aminomethyl)-2-imidazolin-hydrochlorid
62 1-n-Propyl-2-(2,3-dimethylphenyl-aminomethyl)-2-imidazolin-hydrochlorid
63 1-iso-Propyl-2-(2,3-dimethylphenyl-aminomethyl)-2-imidazolin-hydrochlorid
64 1-Ethyl-2-(2-chlor-3-methylphenyl-aminomethyl)-2-imidazolin-hydrochlorid
65 1-Ethyl-2-(3-chlor-2-methylphenyl-aminomethyl)-2-imidazolin-hydrochlorid
66 1-Ethyl-2-(2,3-dichlorphenyl-aminomethyl)-2-imidazolin-hydrochlorid
67 1-Allyl-2-(2,3-dimethylphenyl-aminomethyl)-2-imidazolin-hydrochlorid
68 1-But-2-enyl-2-(2,3-dimethylphenyl-aminomethyl)-2-imidazolin-hydrochlorid
69 1-Propargyl-2-(2,3-dimethylphenyl-aminomethyl)-2-imidazolin-hydrochlorid
70 1-Cyclopropyl-2-(2,3-dimethylphenyl-aminomethyl)-2-imidazolin-hydrochlorid
71 1-Cyclohexyl-2-(2,3-dimethylphenyl-aminomethyl)-2-imidazolin-hydrochlorid
72 1-Cyclopent-2-enyl-2-(2,3-dimethylphenyl-aminomethyl)-2-imidazolin-hydrochlorid
73 1-(2,2,2-Trifluorethyl)-2-(2,3-dimethylphenyl-aminomethyl)-2-imidazolin-hydrochlorid
74 1-(2-Methoxyethyl)-2-(2,3-dimethylphenyl-aminomethyl)-2-imidazolin-hydrochlorid
75 1-Allyl-2-(2,3-dimethylphenoxy-methyl)-2-imidazolin-hydrochlorid
76 1-Propargyl-2-(2,3-dimethylphenoxy-methyl)-2-imidazolin-hydrochlorid
77 1-Cyclopropyl-2-(2,3-dimethylphenoxy-methyl)-2-imidazolin-hydrochlorid
78 1-Cyclopentyl-2-(2,3-dimethylphenoxy-methyl)-2-imidazolin-hydrochlorid
79 1-Cyclopent-2-en-2-(2,3-dimethylphenoxy-methyl)-2-imidazolin-hydrochlorid
80 1-(2,2,2-Trifluorethyl)-2-(2,3-dimethylphenoxy-methyl)-2-imidazolin-hydrochlorid
81 1-(2-Methoxyethyl)-2-(2,3-dimethylphenoxy-methyl)-2-imidazolin-hydrochlorid
82 2-(2,3-Dimethylphenyl-N-ethyl-aminomethyl)-2-imidazolin-hydrochlorid
83 2-(2,3-Dimethylphenyl-N-n-propyl-aminomethyl)-2-imidazolin-hydrochlorid
84 2-(2,3-Dimethylphenyl-N-iso-propyl-aminomethyl)-2-imidazolin-hydrochlorid
85 2-(2,3-Dimethylphenyl-N-butyl-aminomethyl)-2-imidazolin-hydrochlorid
86 2-(2,3-Dimethylphenyl-N-allyl-aminomethyl)-2-imidazolin-hydrochlorid
87 2-(2,3-Dimethylphenyl-N-propargyl-aminomethyl)-2-imidazolin-hydrochlorid
88 2-(2,3-Dimethylphenyl-N-cyclopropyl-aminomethyl)-2-imidazolin-hydrochlorid
89 2-(2,3-Dimethylphenyl-N-cyclopent-2-enyl-aminomethyl)-2-imidazolin-hydrochlorid
90 2-(2,3-Dimethylphenyl-N-(2,2,2-trifluorethyl)-aminomethyl)-2-imidazolin-hydrochlorid
91 2-(2,3-Dimethylphenyl-N-(2-methoxyethyl)-aminomethyl)-2-imidazolin-hydrochlorid
92 1-Ethyl-2-(2,3-dimethylphenyl-N-methyl-aminomethyl)-2-imidazolin-hydrochlorid
93 1-Ethyl-2-(2-chlor-3-methylphenyl-N-methyl-aminomethyl)-2-imidazolin-hydrochlorid
94 1-Ethyl-2-(3-chlor-2-methylphenyl-N-methyl-aminomethyl)-2-imidazolin-hydrochlorid
95 1-Ethyl-2-(2,3-dichlorphenyl-N-methyl-aminomethyl)-2-imidazolin-hydrochlorid
96 1-Ethyl-2-(2,3-dimethylphenyl-N-ethyl-aminomethyl)-2-imidazolin-hydrochlorid
97 2-(2,3,6-Trimethylphenyl-aminomethyl)-2-imidazolin-hydrochlorid
98 2-(2,3-Dimethylphenyl-aminomethyl)-2-imidazolin-hydrochlorid
99 2-(2-Chlor-3-methylphenyl-aminomethyl)-2-imidazolin-hydrochlorid
100 2-(3-Chlor-2-methylphenyl-aminomethyl)-2-imidazolin-hydrochlorid
101 2-(2,3-Dichlorphenyl-aminomethyl)-2-imidazolin-hydrochlorid
102 1-Methyl-2-(2,3-dimethylphenyl-aminomethyl)-2-imidazolin-hydrojodid
103 2-(2,3-Dimethylphenyl-N-methyl-aminomethyl)-2-imidazolin-hydrochlorid
104 2-(2-Chlor-3-methylphenyl-N-methyl-aminomethyl)-2-imidazolin-hydrochlorid
105 2-(3-Chlor-2-methylphenyl-N-methyl-aminomethyl)-2-imidazolin-hydrochlorid
106 2-(2,3-Dichlorphenyl-N-methyl-aminomethyl)-2-imidazolin-hydrochlorid
107 1-Methyl-2-(2,3-dimethylphenyl-N-methyl-aminomethyl)-2-imidazolin-hydrochlorid
108 2-(2,3-Dimethylphenoxy-methyl)-2-imidazolin-hydro chlorid
109 2-(2-Chlor-3-methylphenoxy-methyl)-2-imidazolin-hydrochlorid
110 2-(3-Chlor-2-methylphenoxy-methyl)-2-imidazolin-hydrochlorid
111 2-(1-(2,3-Dimethylphenoxy)-ethyl)-2-imidazolin-hydrochlorid
112 2-(1-(2,3-Dimethylphenoxy)-propyl)-2-imidazolin-hydrochlorid

113 1-Methyl-2-(2,3-dimethylphenoxy-methyl)-2-imidazolin-hydrochlorid
114 1-Ethyl-2-(2,3-dimethylphenoxy-methyl)-2-imidazolin-hydrochlorid
115 1-Methyl-2-(1-(2,3-dimethylphenoxy)-methyl)-2-imidazolin-hydrochlorid

Bei einer wirtstiersystemischen Anwendung von Mitteln zur Ektoparasitenbekämpfung ist neben einer guten Wirksamkeit auch eine gute Verträglichkeit im Wirtstierorganismus gefordert. In der Literatur sind eine ganze Reihe von Imidazolinen beschrieben, die einen Einfluss auf das Herz/Kreislaufsystem von Säugetieren haben (z. B. GB-PS 1.174.349), unter anderem auch solche, die unter den Anspruch der allgemeinen Formel I fallen. Diese unerwünschte Nebenwirkung limitiert die Dosis bei der erfindungsgemäßen Anwendung von Mitteln, die Verbindungen der allgemeinen Formel I enthalten. Die wirksame Dosis zur Bekämpfung von Ektoparasiten und die beim behandelten Wirtstier verträgliche Dosis liegen häufig nahe beieinander. Es ist daher überraschend und war nicht vorherzusehen, daß Verbindungen der allgemeinen Formel I, bei denen $R^5$ ungleich Wasserstoff ist, einen deutlich besseren therapeutischen Index besitzen als Verbindungen mit $R^5$ gleich Wasserstoff.

Besonders bevorzugt sind daher die Verbindungen 9, 51, 52, 54, 60, 61, 62, 64, 65, 67, 70, 73, 74, 92, 93 und 94, deren andere biologisch verträgliche Säureadditionssalze (nicht Hydrochloride) sowie die jeweilige freie Base.

Die Verbindungen der Formel I sind teilweise neu. Gegenstand der vorliegenden Erfindung sind daher auch die Verbindungen der Formel Ia

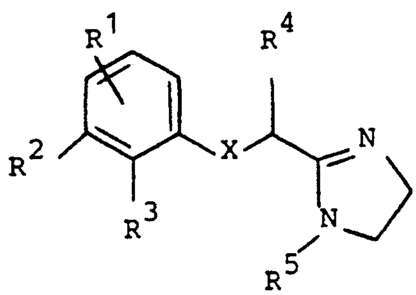

(Ia),

worin

- $R^2$, $R^3$, $R^4$, $R^5$ und $R^6$ die unter Formel I beschriebene Bedeutung haben, wenn X für - $NR^6$- steht und $R^1$ ungleich Wasserstoff und nicht 6-$CH_3$ ist;
- $R^2$, $R^3$, $R^4$, $R^5$ und $R^6$ die unter Formel I beschriebene Bedeutung haben, wenn X für Sauerstoff steht und $R^1$ ungleich Wasserstoff und nicht Chlor oder $CH_3$ ist;
- $R^1$, $R^4$, $R^5$ und $R^6$ die unter Formel I beschriebene Bedeutung haben, wenn X gleich -$NR^6$- ist und $R^2$ und $R^3$ für ($C_2$-$C_5$)-Alkenyl, ($C_2$-$C_5$)-Alkinyl, ($C_3$-$C_7$)-Cycloalkyl, ($C_3$-$C_7$)-Cycloalkenyl, ($C_1$-$C_3$)-Halogenalkyl, Nitro ($C_1$-$C_3$)-Alkoxy-($C_1$-$C_3$)-alkyl, ($C_1$-$C_3$)-Halogenalkoxy oder ($C_1$-$C_3$)-Alkylthio stehen oder zusammen eine Polymethylenkette mit 2 bis 5 C-Atomen bilden;
- $R^1$, $R^4$, $R^5$ und $R^6$ die unter Formel I beschriebene Bedeutung haben, wenn X gleich Sauerstoff ist und $R^2$ und $R^3$ für ($C_2$-$C_5$)-Alkenyl, ($C_2$-$C_5$)-Alkinyl, ($C_3$-$C_7$)-Cycloalkyl, ($C_3$-$C_7$)-Cycloalkenyl, ($C_1$-$C_3$)-Halogenalkyl, Cyano, ($C_1$-$C_3$)-Alkoxy-($C_1$-$C_3$)-alkyl, ($C_1$-$C_3$)-Halogenalkoxy oder ($C_1$-$C_3$)-Alkylthio steht;
- $R^1$, $R^4$, $R^5$ und $R^6$ die unter Formel I beschriebene Bedeutung haben, wenn X für -$NR^6$-steht und mindestens einer der Reste $R^2$ oder $R^3$ ungleich Methyl, Chlor, Cyano oder ($C_1$-$C_5$)-Alkoxy ist;
- $R^1$, $R^4$, $R^5$ und $R^6$ die unter Formel I beschriebene Bedeutung haben, wenn X für Sauerstoff steht und mindestens einer der Reste $R^2$ oder $R^3$ ungleich Methyl, iso-Propyl, Chlor, Nitro oder ($C_1$-$C_5$)-Alkoxy ist oder $R^2$ und $R^3$ zusammen keine Polymethylenkette mit 3 oder 4 C-Atomen bilden;
- $R^1$, $R^2$, $R^3$, $R^5$, $R^6$ und X die unter Formel I beschriebene Bedeutung haben, wenn $R^4$ für ($C_2$-$C_5$)-Alkenyl, ($C_3$-$C_7$)-Cycloalkyl steht;

$R^1$, $R^2$, $R^3$ und $R^5$ die unter Formel I beschriebene Bedeutung haben, wenn X gleich -$NR^6$- und $R^6$ gleich oder ungleich Wasserstoff ist und $R^4$ für ($C_1$-$C_{10}$)-Alkyl, ($C_2$-$C_5$)-Alkenyl, ($C_3$-$C_7$)-Cycloalkyl steht;

- $R^1$, $R^2$, $R^3$, $R^4$ und $R^5$ die unter Formel I beschriebene Bedeutung haben, wenn X für Schwefel steht;
- $R^1$, $R^2$, $R^3$, $R^4$ und $R^6$ die unter Formel I beschriebene Bedeutung haben, wenn X gleich -$NR^6$- ist und $R^5$ für $C_2$-und $C_3$-Alkyl, ($C_3$-$C_5$)-Alkenyl, ($C_3$-$C_5$)-Alkinyl, ($C_3$-$C_7$)-Cycloalkyl, ($C_3$-$C_7$)-Cycloalkenyl, ($C_1$-$C_3$)-Halogenalkyl oder ($C_1$-$C_3$)-Alkoxy-($C_1$-$C_3$)-alkyl steht;
- $R^1$, $R^2$, $R^3$, $R^4$ und $R^6$ die unter Formel I beschriebene Bedeutung haben, wenn X gleich Sauerstoff ist und $R^5$ für ($C_3$-$C_5$)-Alkenyl, ($C_3$-$C_5$)-Alkinyl, ($C_3$-$C_7$)-Cycloalkyl, ($C_3$-$C_7$)-Cycloalkenyl, ($C_1$-$C_3$)-Halogenalkyl oder ($C_1$-$C_3$)-Alkoxy-($C_1$-$C_3$)-alkyl steht;
- $R^1$, $R^2$, $R^3$; $R^4$ und $R^5$ die unter Formel I beschriebene Bedeutung haben, wenn X gleich -$NR^6$- ist und $R^6$

für $(C_2-C_4)$-Alkyl, $(C_3-C_5)$-Alkenyl, $(C_3-C_5)$-Alkinyl, $(C_3-C_7)$-Cycloalkyl, $(C_3-C_7)$-Cycloalkenyl, $(C_1-C_3)$-Halogenalkyl oder $(C_1-C_3)$-Alkoxy-$(C_1-C_3)$-alkyl steht;
sowie deren biologisch verträgliche Säureadditionssalze. Bevorzugte Säureadditionssalze sind die Hydrohalogenide, insbesondere die Hydrochloride.
Bevorzugt unter den Verbindungen der Formel Ia sind diejenigen mit den Beispielnummern 1 - 96. Von diesen die Verbindungen, bei denen $R^5$ ungleich Wasserstoff ist.
Besonders bevorzugt sind daher die Verbindungen 9, 51, 52, 54, 60, 61, 62, 64, 65, 67, 70, 73, 74, 92, 93 und 94, deren andere biologisch verträglichen Säureadditionssalze sowie die jeweilige freie Base.

Die Herstellung der Verbindungen der Formel I ist bekannt und in dem eingangs erwähnten Stand der Technik ausführlich beschrieben. Die neuen Verbindungen der Formel Ia können analog dazu hergestellt werden.
Gegenstand der vorliegenden Erfindung ist somit auch ein Verfahren zur Herstellung der Verbindungen der Formel Ia, dadurch gekennzeichnet, daß man

a) eine Arylverbindung der Formel II mit einem Chloralkyl-imidazolin der Formel III umsetzt, wobei die Reste $R^1$ bis $R^5$ und X die unter Formel Ia festgelegte Bedeutung haben,

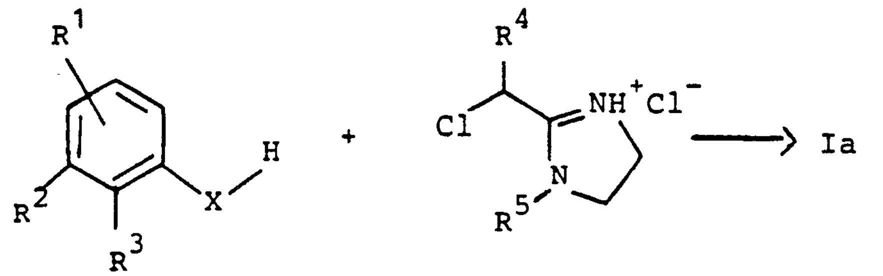

II III

oder

b) eine Arylverbindung der allgemeinen Formel V, wobei Y eine Carboxylgruppe oder ein reaktionsfähiges Derivat derselben, z.B. ein Imidat, Thioimidat, Ester, Orthoester, Thioamid, Imidohalgenid oder Amidin, ist und die Reste $R^1$ bis $R^5$ und X die oben festgelegte Bedeutung haben, mit einem Ethylendiamin der Formel IV oder einem Salz desselben umsetzt

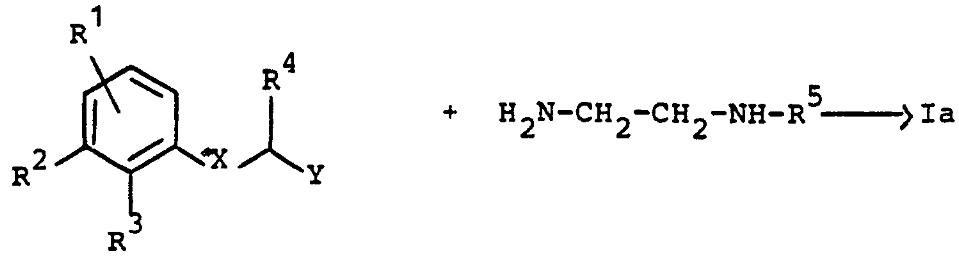

V IV

oder

c) ein Imidazolin der allgemeinen Formel VI mit einem Alkylhalogenid der allgemeinen Formel $R^5$-Z umsetzt, wobei die Reste $R^1$ bis $R^5$ und X die unter Formel Ia festgelegte Bedeutung haben und Z ein Halogenatom ist.

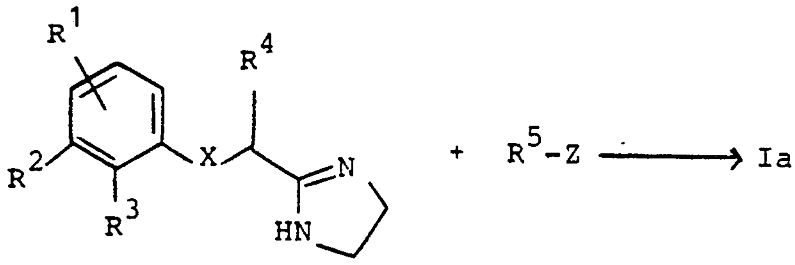

VI

Die Chloralkyl-imidazoline der allgemeinen Formel III lassen sich aus 2-Chloralkansäurederivaten, beispielsweise Imidsäureestern, und einem geeigneten substituierten Ethylendiamin der Formel IV herstellen. (siehe zitierte Literatur).

Die Verbindungen der allgemeinen Formel Ia können aus dem Reaktionsgemisch als freie Base oder in Form eines Säureadditionssalzes isoliert werden. Die Basen können nach an sich bekannten Verfahren in die Säureadditionsalze überführt werden. Dazu werden die Basen in einem Lösungsmittel mit der entsprechenden Säure versetzt. Aus dieser Lösung kristallisieren die Salze entweder direkt oder nach Zugabe eines unpolareren Lösungsmittels oder nach Einengen im Vakuum aus. Die Salze der Verbindungen können ebenso in die freie Base oder in andere Säureadditionssalze überführt werden. Dazu werden die Salze entweder in einem unpolaren Lösungsmittel mit einer organischen Base, z. B. Triethylamin, versetzt und die entstandene konjugierte Säure der eingesetzten Base abgesaugt, wobei die freie Base der Verbindungen der allgemeinen Formel Ia in Lösung bleibt. Oder aber man versetzt eine wäßrige Lösung der Salze mit einem Alkalihydroxid und isoliert die freie Base von Verbindungen der allgemeinen Formel Ia durch Extraktion.

Gegenstand der vorliegenden Erfindung ist ebenfalls ein Verfahren zur Bekämpfung von Ektoparasiten, dadurch gekennzeichnet, daß man dem Wirtstier parenteral oder oral ein eine Verbindung der Formel I enthaltendes Mittel appliziert.
Das Mittel kann auch lediglich aus einer Verbindung der Formel I oder Mischungen aus diesen Verbindungen bestehen.

Bei der wirtstiersystemischen Bekämpfung von Ektoparasiten wird den üblicherweise als Wirt für Ektoparasiten dienenden Säugetieren, wie z.B. Schafen, Rindern oder Hunden, eine geeignete Zubereitung einer Verbindung der allgemeinen Formel I verabreicht. In der Folge lassen die Ektoparasiten vom Wirtstier los, stellen ihre Entwicklung ein oder sterben ab. Beim prophylaktischen Einsatz der Mittel, die Verbindungen der allgemeinen Formel I enthalten, wird ein Befall der Wirtstiere mit Ektoparasiten verhindert oder reduziert.

Bekämpft werden können parasitische Arthropoden der Klasse Acari. Besonders bewährt haben sich die Verbindungen gegen Schildzecken z.B. Ixodes spp., Boophilus spp. z.B. Boophilus microplus, Amblyomma spp., Hyalomma spp., Rhipicephalus spp. z.B. Rhipicephalus appendiculatus, Haemaphysalis spp., Dermacentor spp. und gegen Lederzecken z.B. Ornithodorus spp. wie z.B. Ornithodorus moubata. Außerdem können parasitische Milben z.B. der Gattungen Sarcoptes, Psoroptes und Chorioptes bekämpft werden.

Mittel, die Verbindungen der allgemeinen Formel I enthalten, können den Wirtstieren entweder parenteral, percutan oder oral gegeben werden. Die Verabreichung kann z.B. als subcutane, intravenöse, intramuskuläre oder intraperitoneale Injektion erfolgen. Bei oraler Anwendung kann das Mittel entweder als solches oder mit geeigneten Hilfs- oder Verdünnungsstoffen oder als Mischung mit dem Futter verabreicht werden. Besonders bevorzugte Applikationsmethoden sind die subcutane Injektion und die orale Gabe mit dem Futter.

Um eine gute Dauerwirkung zu erreichen, kann eine Verbindung der allgemeinen Formel I mehrfach verabreicht werden oder es kann eine solche Verbindung verwendet werden, die eine besonders günstige Halbwertszeit im Wirtstierorganismus aufweist oder es kann eine Depotzubereitung oder ein Applikationssystem zur Niedrigdauerdosierung des Wirkstoffes, wie z.B. ein Implantat oder ein Bolus, eingesetzt werden. In der Praxis hat sich eine Kombination dieser Maßnahmen bewährt.

Mittel, die zur topicalen Bekämpfung von Ektoparasiten eingesetzt werden, enthalten neben dem eigentlichen Wirkstoff in der Regel andere Hilfs- und Zuschlagstoffe als solche Mittel, die oral oder parenteral bei Säugetieren angewendet werden. Während erstere sich in der Zusammensetzung an den

üblichen Formulierungen fuer Pflanzenschutzmittel orientieren, sind letztere pharmazeutische Zubereitungen. Gegenstand der Erfindung sind daher auch Tierarzneimittel, die eine oder mehrere Verbindungen der allgemeinen Formel I enthalten, und die speziell zur oralen oder parenteralen Anwendung bei Vertebraten, insbesondere bei in der Landwirtschaft wichtigen Nutztieren und hier speziell bei Rindern, Schafen und Schweinen, sowie Haustieren, wie Hunden und Katzen, geeignet sind.

Zur oralen Applikation kommen z.B. Pulver, Tabletten, Boli, Kapseln, Granulate, Lösungen, Suspensionen, Emulsionen oder Pasten in Betracht, welche die Wirkstoffe allein oder zusammen mit üblichen Hilfs- und Trägerstoffen wie Stärke, Cellulosepulver, Talcum, Magnesiumstearat, Zucker, Gelatine, feinverteilter Kieselsäure, Carboxymethylcellulose oder ähnlichen Stoffen enthalten. Zur Niedrigdauerdosierung eignen sich dabei besonders gut Boli, aus denen Verbindungen der allgemeinen Formel I z.B. im Pansen von Rindern nur langsam freigesetzt werden.

Zur parenteralen Applikation kommen z.B. Lösungen, Suspensionen, Emulsionen in Betracht, die aus den Wirkstoffen unter Verwendung von üblichen Lösungsmitteln unter Zusatz von geeigneten Hilfs- und Trägerstoffen hergestellt werden. Verwendet werden können z.B. wäßrige Lösungen, die ggf. Verdickungsmittel, wie z.B. Polyethylenglykol oder Carboxymethylcellulose, enthalten, oder aber ölige Suspensionen, die unter Verwendung von z.B. Sesamöl, Olivenöl oder synthetischen Triglyceriden, ggf. unter Verwendung von grenzflächenaktiven Stoffen wie Sorbitanfettsäureestern, hergestellt werden. Zur Niedrigdauerdosierung bei parenteraler Applikation eignen sich insbesondere Implantate, bei denen Verbindungen der allgemeinen Formel I in eine biologisch abbaubare Matrix eingebettet sind. Eine solche Matrix kann z.B. ein Stärkegel sein, das mit Glycerin weichgemacht und geeignet geformt ist.

Von besonderer Bedeutung für beide Applikationstypen sind Depotzubereitungen (vgl. DE-OS 3 521 893 und 3 523 065, EP-A 0 164 927)

Die Wirkstoffkonzentration der Verbindungen I betragen allgemein

a) im Futter : 100 - 10.000 ppm, bevorzugt 500 - 5.000 ppm;

b) als Injektionslösung : 1 - 50 Gew.-%, bevorzugt 10 -30 Gew.-%;

und

c) als Depotzubereitung ("Slow-Release") : 5 - 90 Gew.-%, bevorzugt 10 - 50 Gew.-%.

Allgemein ist die Dosis so zu wählen, daß die Verbindungen I in einer Konzentration von ca. 0,1 - 20 mg/kg Körpergewicht des Wirtstieres, bevorzugt 0,5 - 10 mg/kg Körpergewicht, vorliegen.

## A. Chemische Beispiele

### Herstellungsvorschriften

a) 2-(3-Chlor-2-methylphenyl-thiomethyl)-2-imidazolin-hydrochlorid (56)

5.55 g (35 mmol) 3-Chlor-2-methyl-thiophenol, 5.43 g (35 mmol) 2-Chlormethyl-2-imidazolin-hydrochlorid und 5 g Kaliumcarbonat werden bei Raumtemperatur in 40 ml Ethanol gerührt. Nach 25 h filtriert man und engt das Filtrat zur Trockene ein. Das Rohprodukt wird durch Säulenchromatographie an Kieselgel gereinigt. Nach dem Abziehen des Elutionsmittels wird der Rückstand mit ethanolischer Salzsäure versetzt und das Produkt durch Zugabe von Ether ausgefällt. Man erhält weiße Kristalle mit Fp. 208 -209 °C.

b) 1-Ethyl-2-(2,3-dimethylphenyl-aminomethyl)-2-imidazolin-hydrochlorid (60)

19,4 g (0.16 mol) 2,3-Dimethylanilin und 14,6 g (0.08 mol) 1-Ethyl-2-chlormethyl-2-imidazolin-hydrochlorid werden in 50 ml Ethanol 3 h zum Rückfluß erhitzt. Danach zieht man das Lösungsmittel im Vakuum weitgehend ab, filtriert den erhaltenen Kristallbrei und kristallisiert den Filterkuchen mehrfach aus Isopropanol um. Ausbeute: 16.5 g (78 % d.Th.) weiße Kristalle mit Fp. = 209 - 211 °C

c) 1-n-Propyl-2-(2,3-dimethylphenyl-aminomethyl)-2-imidazolin-hydrochlorid (62)

4.8 g (30 mmol) 2,3-Dimethylanilino-acteonitril, 3.4 g (33 mmol) N-(n-Propyl)-ethylendiamin und 260 mg Natriumpolysulfid (Na2S4) werden zusammen 4 h bei 50 °C gerührt. Dann zieht man das überschüßige

Amin im Hochvakuum ab, versetzt den Rückstand mit ethanolischer Salzsäure und fällt das Hydrochlorid durch Zugabe von Ether aus. Das Rohprodukt wird durch Umkristallisation aus Aceton/Ether gereinigt. Man erhält 5.7 g (67 %) mit Fp. 145 - 148 °C.

d) 1-Allyl-2-(2,3-dimethylphenoxy-methyl)-2-imidazolin-hydrochlorid (75)

4.84 g (30 mmol) 2,3-Dimethylphenoxy-acetonitril, 3.3 g (33 mmol) N-Allyl-ethylendiamin und 260 mg Natriumpolysulfid (Na2S4) werden zusammen 4 h bei 50 °C gerührt. Dann zieht man das überschüssige Amin im Hochvakuum ab, versetzt den Rückstand mit ethanolischer Salzsäure und fällt das Hydrochlorid durch Zugabe von Ether aus.

Gemäß diesen Vorschriften lassen sich die Verbindungen der allgemeinen Formel I herstellen.

| Nr. | $R^1$ | $R^2$ | $R^3$ | X | $R^4$ | $R^5$ | $F_p$[°C] |
|---|---|---|---|---|---|---|---|
| 1 | $CF_3$ | Cl | 5-Cl | NH | H | H | 288 (Zers.) |
| 2 | $CH_3$ | Cl | 6-Cl | NH | H | H | 263 - 266 |
| 4 | $CH_3$ | Cl | 4-F | NH | H | H | 267 (Zers.) |
| 5 | $CF_3$ | Cl | 4-F | NH | H | H | 279 - 283 |
| 6 | F | Cl | 4-Cl | NH | H | H | 291 |
| 7 | $CH_3$ | $CH_3$ | 4-$OCH_3$ | NH | H | H | 233 - 237 |
| 8 | $CH_3$ | $CH_3$ | 4-$CH_3$ | NH | H | H | 244 |
| 19 | $NO_2$ | $CH_3$ | H | NH | H | H | 254 (Zers.) |
| 28 | $-(CH_2)_4-$ | | H | NH | H | H | 243 - 246 |
| 39 | F | $CH_3$ | H | NH | H | H | 261 (Zers.) |
| 40 | Br | $CH_3$ | H | NH | H | H | 271 (Zers.) |
| 41 | Cl | F | H | NH | H | H | 267 - 271 |
| 49 | $CH_3$ | $CH_3$ | H | NH | $CH_3$ | H | 241 - 243 |
| 52 | $CH_3$ | $CH_3$ | H | NH | $CH_3$ | $C_2H_5$ | 148 - 150 |
| 56 | Cl | $CH_3$ | H | S | H | H | 208 - 209 |
| 60 | $CH_3$ | $CH_3$ | H | NH | H | $C_2H_5$ | 209 - 211 |
| 62 | $CH_3$ | $CH_3$ | H | NH | H | n-Prop | 145 - 148 |
| 63 | $CH_3$ | $CH_3$ | H | NH | H | i-Prop | 198 (Zers.) |
| 67 | $CH_3$ | $CH_3$ | H | NH | H | Allyl | 167 - 171 |
| 71 | $CH_3$ | $CH_3$ | H | NH | H | 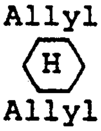 | 70 - 78 |
| 75 | $CH_3$ | $CH_3$ | H | O | H | Allyl | 170 - 173 |
| 92 | $CH_3$ | $CH_3$ | H | $NCH_3$ | H | $C_2H_5$ | |
| 66 | Cl | Cl | H | NH | H | $C_2H_5$ | 284 - 287 |
| 73 | $CH_3$ | $CH_3$ | H | NH | H | $CH_2CF_3$ | 207 - 210 |

B. Biologische Beispiele

Als Testmodell zum Nachweis einer tiersystemischen ektoparasitiziden Wirksamkeit dienen Meerschweinchen mit Zeckeninfestation.

Dazu werden Meerschweinchen in Plastikzylindern sitzend am Tag d 0 mit jeweils 100 nüchternen Nymphen der Braunen Hundezecke, Ripicephalus sanguineus, infestiert. Am Tag d + 1, wenn sich die Zecken festgesaugt haben, werden die Meerschweinchen in eine Apparatur verbracht, die das Auffangen der im vollgesogenen Zustand losgelassenen Nymphen gestattet. Bei unbehandelten Kontrolltieren sind die Zecken im Verlauf der Tage d + 5, d + 6 und d + 7 vollgesogen, lassen vom Wirtstier los und können aus der Apparatur entnommen werden. Im allgemeinen gelangen unter diesen Bedingungen auf den Meerschweinchen 20-50 der 100 Nymphen zur Entwicklung.

Zur Prüfung der tiersystemischen Wirksamkeit werden Substanzen der allgemeinen Formel I den zeckenbesetzten Meerschweinchen an den Tagen d + 1, d + 2, d + 3 und d + 4 in einer Dosierung von jeweils 10 mg/kg und weniger subcutan injiziert. Wenn die Prüfsubstanz tiersystemisch wirksam ist, gelangen im Gegensatz zu den unbehandelten Kontrollen keine oder nur wenige Zecken zur Entwicklung.

In diesem Test wurden mit der Dosierung 10 mg/kg die in der Tabelle enthaltenen Ergebnisse erzielt:

| Substanz-Nr. | Zahl der Zecken entwickelt |
|---|---|
| 19 | 2 |
| 60 | 0 |
| 61 | 1 |
| 98 | 0 |
| 99 | 0 |
| 100 | 0 |
| 101 | 0 |
| 102 | 0 |
| 103 | 0 |
| 106 | 0 |
| 108 | 0 |

## Ansprüche

1. Mittel zur wirtsstiersystemischen Bekämpfung von Ektoparasiten, dadurch gekennzeichnet, daß sie eine Verbindung der Formel I

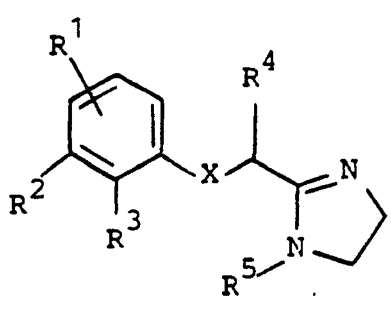

(I),

worin

$R^1$ Wasserstoff, $(C_1\text{-}C_5)$-Alkyl, $(C_1\text{-}C_3)$-Halogenalkyl oder Halogen;

$R^2$, $R^3$ unabhängig voneinander $(C_1\text{-}C_5)$-Alkyl, $(C_2\text{-}C_5)$Alkenyl, $(C_2\text{-}C_5)$-Alkinyl, $(C_3\text{-}C_7)$-Cycloalkyl, $(C_3\text{-}C_7)$-Cycloalkenyl, $(C_1\text{-}C_3)$-Halogenalkyl, Halogen Cyano, Nitro, $(C_1\text{-}C_5)$-Alkoxy, $(C_1\text{-}C_3)$-Alkoxy-$(C_1\text{-}C_3)$-alkyl, $(C_1\text{-}C_3)$-Halogenalkoxy, $(C_1\text{-}C_3)$-Alkylthio,

oder $R^2$ und $R^3$ bilden zusammen eine Polymethylenkette mit 2 bis 5 C-Atomen;

$R^4$ Wasserstoff, $(C_1\text{-}C_{10})$-Alkyl, $(C_2\text{-}C_5)$-Alkenyl, $(C_3\text{-}C_7)$-Cycloalkyl;

$R^5$ Wasserstoff, $(C_1\text{-}C_{10})$-Alkyl, $(C_3\text{-}C_5)$-Alkenyl, $(C_3\text{-}C_5)$-Alkinyl, $(C_3\text{-}C_7)$-Cycloalkyl, $(C_3\text{-}C_7)$-Cycloalkenyl, $(C_1\text{-}C_3)$-Halogenalkyl oder $(C_1\text{-}C_3)$-Alkoxy-$(C_1\text{-}C_3)$-alkyl,

X Sauerstoff, Schwefel oder eine -$NR^6$-Gruppe;

$R^6$ Wasserstoff, $(C_1\text{-}C_5)$-Alkyl, $(C_3\text{-}C_5)$-Alkenyl, $(C_3\text{-}C_5)$-Alkinyl, $(C_3\text{-}C_7)$-Cycloalkyl, $(C_3\text{-}C_7)$-Cycloalkenyl, $(C_1\text{-}C_3)$-Halogenalkyl oder $(C_1\text{-}C_3)$-Alkoxy-$(C_1\text{-}C_3)$-alkyl

bedeuten, oder deren biologisch verträgliche Säureadditionssalze enthalten.

2. Mittel zur wirtstiersystemischen Bekämpfung von Ektoparasiten gemäß Anspruch 1, dadurch gekennzeichnet, daß in Formel I

$R^1$ Wasserstoff, $(C_1\text{-}C_3)$-Alkyl, $(C_1\text{-}C_3)$-Halogenalkyl, F oder Cl;

$R^2$, $R^3$ unabhängig voneinander $(C_1\text{-}C_3)$-Alkyl, $(C_2\text{-}C_3)$-Alkenyl, $(C_2\text{-}C_3)$-Alkinyl, $(C_3\text{-}C_5)$-Cycloalkyl, $(C_1\text{-}C_3)$-Halogenalkyl, Halogen, Cyano, Nitro, $(C_1\text{-}C_3)$-Alkoxy, $(C_1\text{-}C_3)$-Alkoxy-$(C_1\text{-}C_2)$-alkyl, $(C_1\text{-}C_3)$-Halogenalkoxy, $(C_1\text{-}C_3)$-Alkylthio, oder $R^2$ und $R^3$ bilden zusammen eine Polymethylenkette mit 2 bis 5 C-Atomen;

$R^4$ Wasserstoff, $(C_1\text{-}C_4)$-Alkyl, $(C_2\text{-}C_3)$-Alkenyl, $(C_3\text{-}C_6)$-Cycloalkyl;

$R^5$ Wasserstoff, $(C_1\text{-}C_4)$-Alkyl, $(C_3\text{-}C_4)$-Alkenyl, $(C_3\text{-}C_4)$-Alkinyl, $(C_3\text{-}C_6)$-Cycloalkyl, $(C_3\text{-}C_5)$-Cycloalkenyl, $(C_1\text{-}C_3)$-Halogenalkyl oder $(C_1\text{-}C_3)$-Alkoxy-$(C_1\text{-}C_2)$-alkyl

X Sauerstoff oder eine -$NR^6$-Gruppe;

$R^6$ Wasserstoff, $(C_1\text{-}C_4)$-Alkyl, $(C_3\text{-}C_4)$-Alkenyl, $(C_3\text{-}C_4)$-Alkinyl, $(C_3\text{-}C_6)$-Cycloalkyl, $(C_1\text{-}C_3)$-Halogenalkyl oder $(C_1\text{-}C_3)$-Alkoxy-$(C_1 - C_2)$-alkyl

24

bedeuten, sowie deren biologisch verträgliche Säureadditionssalze.

3. Verbindungen der Formel Ia

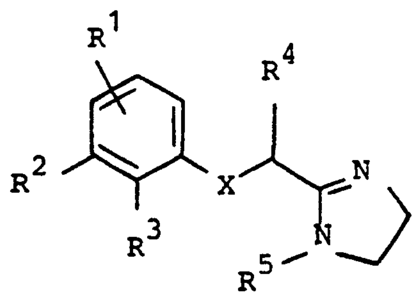

(Ia),

worin

- $R^2$, $R^3$, $R^4$, $R^5$ und $R^6$ die unter Formel I von Anspruch 1 beschriebene Bedeutung haben, wenn X für -$NR^6$- steht und $R^1$ ungleich Wasserstoff und nicht 6-$CH_3$ ist;
- $R^2$, $R^3$, $R^4$, $R^5$ und $R^6$ die unter Formel I beschriebene Bedeutung haben, wenn X für Sauerstoff steht und $R^1$ ungleich Wasserstoff und nicht Chlor oder $CH_3$ ist;
- $R^1$, $R^4$, $R^5$ und $R^6$ die unter Formel I beschriebene Bedeutung haben, wenn X gleich -$NR^6$- ist und $R^2$ und $R^3$ für $(C_2\text{-}C_5)$-Alkenyl, $(C_2\text{-}C_5)$-Alkinyl, $(C_3\text{-}C_7)$-Cycloalkyl, $(C_3\text{-}C_7)$-Cycloalkenyl, $(C_1\text{-}C_3)$-Halogenalkyl, Nitro $(C_1\text{-}C_3)$-Alkoxy-$(C_1\text{-}C_3)$-alkyl, $(C_1\text{-}C_3)$-Halogenalkoxy oder $(C_1\text{-}C_3)$-Alkylthio stehen oder zusammen eine Polymethylenkette mit 2 bis 5 C-Atomen bilden;
- $R^1$, $R^4$, $R^5$ und $R^6$ die unter Formel I beschriebene Bedeutung haben, wenn X gleich Sauerstoff ist und $R^2$ und $R^3$ für $(C_2\text{-}C_5)$-Alkenyl, $(C_2\text{-}C_5)$-Alkinyl, $(C_3\text{-}C_7)$-Cycloalkyl, $(C_3\text{-}C_7)$-Cycloalkenyl, $(C_1\text{-}C_3)$-Halogenalkyl, Cyano, $(C_1\text{-}C_3)$-Alkoxy-$(C_1\text{-}C_3)$-alkyl, $(C_1\text{-}C_3)$-Halogenalkoxy oder $(C_1\text{-}C_3)$-Alkylthio steht;
- $R^1$, $R^4$, $R^5$ und $R^6$ die unter Formel I beschriebene Bedeutung haben, wenn X für -$NR^6$-steht und mindestens einer der Reste $R^2$ oder $R^3$ ungleich Methyl, Chlor, Cyano oder $(C_1\text{-}C_5)$-Alkoxy ist;
- $R^1$, $R^4$, $R^5$ und $R^6$ die unter Formel I beschriebene Bedeutung haben, wenn X für Sauerstoff steht und mindestens einer der Reste $R^2$ oder $R^3$ ungleich Methyl, iso-Propyl, Chlor, Nitro oder $(C_1\text{-}C_5)$-Alkoxy ist oder $R^2$ und $R^3$ zusammen keine Polymethylenkette mit 3 oder 4 C-Atomen bilden;
- $R^1$, $R^2$, $R^3$, $R^5$, $R^6$ und X die unter Formel I beschriebene Bedeutung haben, wenn $R^4$ für $(C_2\text{-}C_5)$-Alkenyl, $(C_3\text{-}C_7)$-Cycloalkyl steht;

$R^1$, $R^2$, $R^3$ und $R^5$ die unter Formel I beschriebene Bedeutung haben, wenn X gleich -$NR^6$- und $R^6$ gleich oder ungleich Wasserstoff ist und $R^4$ für $(C_1\text{-}C_{10})$-Alkyl, $(C_2\text{-}C_5)$-Alkenyl, $(C_3\text{-}C_7)$-Cycloalkyl steht;

- $R^1$, $R^2$, $R^3$, $R^4$ und $R^5$ die unter Formel I beschriebene Bedeutung haben, wenn X für Schwefel steht;
- $R^1$, $R^2$, $R^3$, $R^4$ und $R^6$ die unter Formel I beschriebene Bedeutung haben, wenn X gleich -$NR^6$- ist und $R^5$ für $C_2$-und $C_3$-Alkyl, $(C_3\text{-}C_5)$-Alkenyl, $(C_3\text{-}C_5)$-Alkinyl, $(C_3\text{-}C_7)$-Cycloalkyl, $(C_3\text{-}C_7)$-Cycloalkenyl, $(C_1\text{-}C_3)$-Halogenalkyl oder $(C_1\text{-}C_3)$-Alkoxy-$(C_1\text{-}C_3)$-alkyl steht;
- $R^1$, $R^2$, $R^3$, $R^4$ und $R^6$ die unter Formel I beschriebene Bedeutung haben, wenn X gleich Sauerstoff ist und

$R^5$ für $(C_3-C_5)$-Alkenyl, $(C_3-C_5)$-Alkinyl, $(C_3-C_7)$-Cycloalkyl, $(C_3-C_7)$-Cycloalkenyl, $(C_1-C_3)$-Halogenalkyl oder $(C_1-C_3)$-Alkoxy-$(C_1-C_3)$-alkyl steht;
- $R^1$, $R^2$, $R^3$, $R^4$ und $R^5$ die unter Formel I beschriebene Bedeutung haben, wenn X gleich $-NR^6-$ ist und $R^6$ für $(C_2-C_4)$-Alkyl, $(C_3-C_5)$-Alkenyl, $(C_3-C_5)$-Alkinyl, $(C_3-C_7)$-Cycloalkyl, $(C_3-C_7)$-Cycloalkenyl, $(C_1-C_3)$-Halogenalkyl oder $(C_1-C_3)$-Alkoxy-$(C_1-C_3)$-alkyl steht;
sowie deren biologisch verträgliche Säureadditionssalze.

4. Verfahren zur Herstellung von Verbindungen der Formel Ia gemäß Anspruch 3, dadurch gekennzeichnet, daß man

a) eine Arylverbindung der Formel II mit einem Chloralkyl-imidazolin der Formel III umsetzt, wobei die Reste $R^1$ bis $R^5$ und X die unter Formel Ia festgelegte Bedeutung haben,

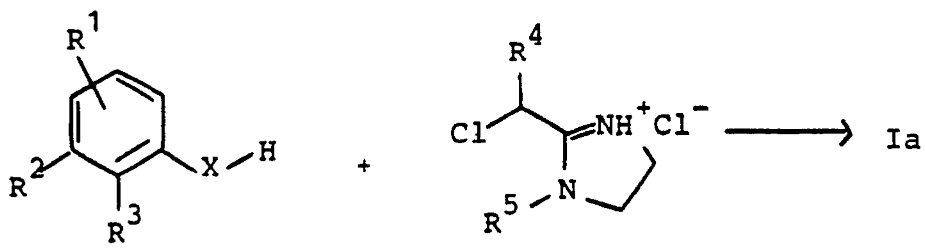

II III

oder

b) eine Arylverbindung der allgemeinen Formel V, wobei Y eine Carboxylgruppe oder ein reaktionsfähiges Derivat derselben, z.B. ein Imidat, Thioimidat, Ester, Orthoester, Thioamid, Imidohalgenid oder Amidin, ist und die Reste $R^1$ bis $R^5$ und X die oben festgelegte Bedeutung haben, mit einem Ethylendiamin der Formel IV oder einem Salz desselben umsetzt

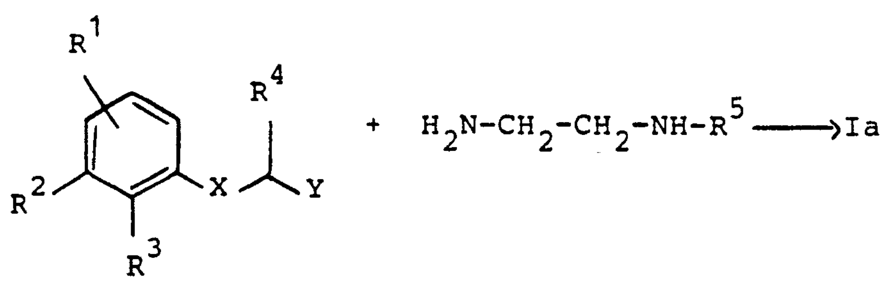

V IV

oder

c) ein Imidazolin der allgemeinen Formel VI mit einem Alkylhalogenid der allgemeinen Formel $R^5$-Z umsetzt, wobei die Reste $R^1$ bis $R^5$ und X die unter Formel Ia festgelegte Bedeutung haben und Z ein Halogenatom ist.

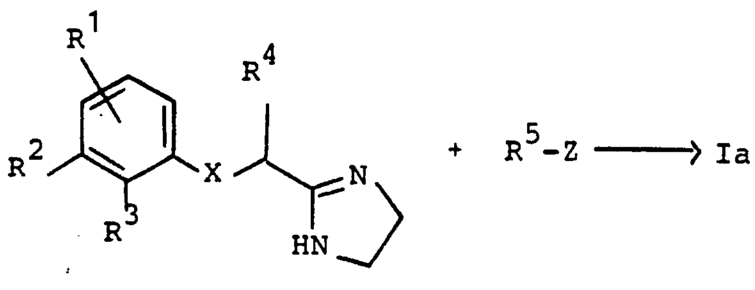

VI

5. Tierarzneimittel, dadurch gekennzeichnet, daß es eine Verbindung der Formel I nach Anspruch 1 enthält oder aus ihr besteht.

6. Verwendung von Verbindungen der Formel I nach Anspruch 1 zur wirtstiersystemischen Bekämpfung von Ektoparasiten.

Patentansprüche für folgenden Vertragsstaat: ES

1. Mittel zur wirtsstiersystemischen Bekämpfung von Ektoparasiten, dadurch gekennzeichnet, daß sie eine Verbindung der Formel I

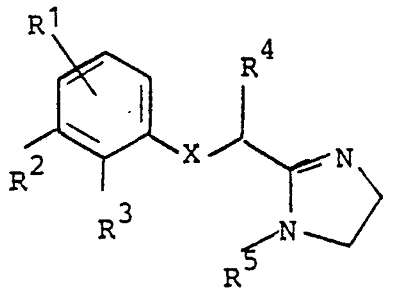

(I) ,

worin
$R^1$ Wasserstoff, $(C_1-C_5)$-Alkyl, $(C_1-C_3)$-Halogenalkyl oder Halogen;
$R^2$, $R^3$ unabhängig voneinander $(C_1-C_5)$-Alkyl, $(C_2-C_5)$Alkenyl, $(C_2-C_5)$-Alkinyl, $(C_3-C_7)$-Cycloalkyl, $(C_3-C_7)$-Cycloalkenyl, $(C_1-C_3)$-Halogenalkyl, Halogen Cyano, Nitro, $(C_1-C_5)$-Alkoxy, $(C_1-C_3)$-Alkoxy-$(C_1-C_3)$-alkyl, $(C_1-C_3)$-Halogenalkoxy, $(C_1-C_3)$-Alkylthio,
oder $R^2$ und $R^3$ bilden zusammen eine Polymethylenkette mit 2 bis 5 C-Atomen;
$R^4$ Wasserstoff, $(C_1-C_{10})$-Alkyl, $(C_2-C_5)$-Alkenyl, $(C_3-C_7)$-Cycloalkyl;
$R^5$ Wasserstoff, $(C_1-C_{10})$-Alkyl, $(C_3-C_5)$-Alkenyl, $(C_3-C_5)$-Alkinyl, $(C_3-C_7)$-Cycloalkyl, $(C_3-C_7)$-Cycloalkenyl, $(C_1-C_3)$-Halogenalkyl oder $(C_1-C_3)$-Alkoxy-$(C_1-C_3)$-alkyl,
X Sauerstoff, Schwefel oder eine -$NR^6$-Gruppe;
$R^6$ Wasserstoff, $(C_1-C_5)$-Alkyl, $(C_3-C_5)$-Alkenyl, $(C_3-C_5)$-Alkinyl, $(C_3-C_7)$-Cycloalkyl, $(C_3-C_7)$-Cycloalkenyl, $(C_1-C_3)$-Halogenalkyl oder $(C_1-C_3)$-Alkoxy-$(C_1-C_3)$-alkyl
bedeuten, oder deren biologisch verträgliche Säureadditionssalze enthalten.

2. Mittel zur wirtstiersystemischen Bekämpfung von Ektoparasiten gemäß Anspruch 1, dadurch gekennzeichnet, daß in Formel I
$R^1$ Wasserstoff, $(C_1-C_3)$-Alkyl, $(C_1-C_3)$-Halogenalkyl, F oder Cl;
$R^2$, $R^3$ unabhängig voneinander $(C_1-C_3)$-Alkyl, $(C_2-C_3)$-Alkenyl, $(C_2-C_3)$-Alkinyl, $(C_3-C_5)$-Cycloalkyl, $(C_1-C_3)$-Halogenalkyl, Halogen, Cyano, Nitro, $(C_1-C_3)$-Alkoxy, $(C_1-C_3)$-Alkoxy-$(C_1-C_2)$-alkyl, $(C_1-C_3)$-Halogenalkoxy, $(C_1-C_3)$-Alkylthio, oder $R^2$ und $R^3$ bilden zusammen eine Polymethylenkette mit 2 bis 5 C-Atomen;
$R^4$ Wasserstoff, $(C_1-C_4)$-Alkyl, $(C_2-C_3)$-Alkenyl, $(C_3-C_6)$-Cycloalkyl;
$R^5$ Wasserstoff, $(C_1-C_4)$-Alkyl, $(C_3-C_4)$-Alkenyl, $(C_3-C_4)$-Alkinyl, $(C_3-C_6)$-Cycloalkyl, $(C_3-C_5)$-Cycloalkenyl, $(C_1-C_3)$-Halogenalkyl oder $(C_1-C_3)$-Alkoxy-$(C_1-C_2)$-alkyl
X Sauerstoff oder eine -$NR^6$-Gruppe;
$R^6$ Wasserstoff, $(C_1-C_4)$-Alkyl, $(C_3-C_4)$-Alkenyl, $(C_3-C_4)$-Alkinyl, $(C_3-C_6)$-Cycloalkyl, $(C_1-C_3)$-Halogenalkyl

oder ($C_1$-$C_3$)-Alkoxy-($C_1$ – $C_2$)-alkyl

24

bedeuten, sowie deren biologisch verträgliche Säureadditionssalze.

3. Verfahren zur Herstellung von Verbindungen der Formel Ia

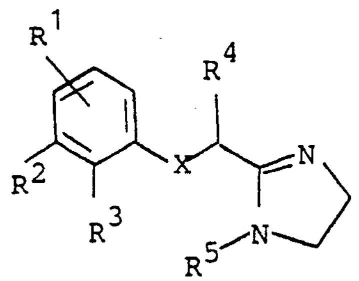

(Ia),

worin

- $R^2$, $R^3$, $R^4$, $R^5$ und $R^6$ die unter Formel I von Anspruch 1 beschriebene Bedeutung haben, wenn X für -$NR^6$- steht und $R^1$ ungleich Wasserstoff und nicht 6-$CH_3$ ist;
- $R^2$, $R^3$, $R^4$, $R^5$ und $R^6$ die unter Formel I beschriebene Bedeutung haben, wenn X für Sauerstoff steht und $R^1$ ungleich Wasserstoff und nicht Chlor oder $CH_3$ ist;
- $R^1$, $R^4$, $R^5$ und $R^6$ die unter Formel I beschriebene Bedeutung haben, wenn X gleich -$NR^6$- ist und $R^2$ und $R^3$ für ($C_2$-$C_5$)-Alkenyl, ($C_2$-$C_5$)-Alkinyl, ($C_3$-$C_7$)-Cycloalkyl, ($C_3$-$C_7$)-Cycloalkenyl, ($C_1$-$C_3$)-Halogenalkyl, Nitro ($C_1$-$C_3$)-Alkoxy-($C_1$-$C_3$)-alkyl, ($C_1$-$C_3$)-Halogenalkoxy oder ($C_1$-$C_3$)-Alkylthio stehen oder zusammen eine Polymethylenkette mit 2 bis 5 C-Atomen bilden;
- $R^1$, $R^4$, $R^5$ und $R^6$ die unter Formel I beschriebene Bedeutung haben, wenn X gleich Sauerstoff ist und $R^2$ und $R^3$ für ($C_2$-$C_5$)-Alkenyl, ($C_2$-$C_5$)-Alkinyl, ($C_3$-$C_7$)-Cycloalkyl, ($C_3$-$C_7$)-Cycloalkenyl, ($C_1$-$C_3$)-Halogenalkyl, Cyano, ($C_1$-$C_3$)-Alkoxy-($C_1$-$C_3$)-alkyl, ($C_1$-$C_3$)-Halogenalkoxy oder ($C_1$-$C_3$)-Alkylthio steht;
- $R^1$, $R^4$, $R^5$ und $R^6$ die unter Formel I beschriebene Bedeutung haben, wenn X für -$NR^6$-steht und mindestens einer der Reste $R^2$ oder $R^3$ ungleich Methyl, Chlor, Cyano oder ($C_1$-$C_5$)-Alkoxy ist;
- $R^1$, $R^4$, $R^5$ und $R^6$ die unter Formel I beschriebene Bedeutung haben, wenn X für Sauerstoff steht und mindestens einer der Reste $R^2$ oder $R^3$ ungleich Methyl, iso-Propyl, Chlor, Nitro oder ($C_1$-$C_5$)-Alkoxy ist oder $R^2$ und $R^3$ zusammen keine Polymethylenkette mit 3 oder 4 C-Atomen bilden;
- $R^1$, $R^2$, $R^3$, $R^5$, $R^6$ und X die unter Formel I beschriebene Bedeutung haben, wenn $R^4$ für ($C_2$-$C_5$)-Alkenyl, ($C_3$-$C_7$)-Cycloalkyl steht;

$R^1$, $R^2$, $R^3$ und $R^5$ die unter Formel I beschriebene Bedeutung haben, wenn X gleich -$NR^6$- und $R^6$ gleich oder ungleich Wasserstoff ist und $R^4$ für ($C_1$-$C_{10}$)-Alkyl, ($C_2$-$C_5$)-Alkenyl, ($C_3$-$C_7$)-Cycloalkyl steht;

- $R^1$, $R^2$, $R^3$, $R^4$ und $R^5$ die unter Formel I beschriebene Bedeutung haben, wenn X für Schwefel steht;
- $R^1$, $R^2$, $R^3$, $R^4$ und $R^6$ die unter Formel I beschriebene Bedeutung haben, wenn X gleich -$NR^6$- ist und $R^5$ für $C_2$-und $C_3$-Alkyl, ($C_3$-$C_5$)-Alkenyl, ($C_3$-$C_5$)-Alkinyl, ($C_3$-$C_7$)-Cycloalkyl, ($C_3$-$C_7$)-Cycloalkenyl, ($C_1$-$C_3$)-Halogenalkyl oder ($C_1$-$C_3$)-Alkoxy-($C_1$-$C_3$)-alkyl steht;
- $R^1$, $R^2$, $R^3$, $R^4$ und $R^6$ die unter Formel I beschriebene Bedeutung haben, wenn X gleich Sauerstoff ist und $R^5$ für ($C_3$-$C_5$)-Alkenyl, ($C_3$-$C_5$)-Alkinyl, ($C_3$-$C_7$)-Cycloalkyl, ($C_3$-$C_7$)-Cycloalkenyl, ($C_1$-$C_3$)-Halogenalkyl oder ($C_1$-$C_3$)-Alkoxy-($C_1$-$C_3$)-alkyl steht;
- $R^1$, $R^2$, $R^3$, $R^4$ und $R^5$ die unter Formel I beschriebene Bedeutung haben, wenn X gleich -$NR^6$- ist und $R^6$ für ($C_2$-$C_4$)-Alkyl, ($C_3$-$C_5$)-Alkenyl, ($C_3$-$C_5$)-Alkinyl, ($C_3$-$C_7$)-Cycloalkyl, ($C_3$-$C_7$)-Cycloalkenyl, ($C_1$-$C_3$)-Halogenalkyl oder ($C_1$-$C_3$)-Alkoxy-($C_1$-$C_3$)-alkyl steht;

sowie deren biologisch verträgliche Säureadditionssalze, dadurch gekennzeichnet, daß man

a) eine Arylverbindung der Formel II mit einem Chloralkyl-imidazolin der Formel III umsetzt, wobei die Reste $R^1$ bis $R^5$ und X die unter Formel Ia festgelegte Bedeutung haben,

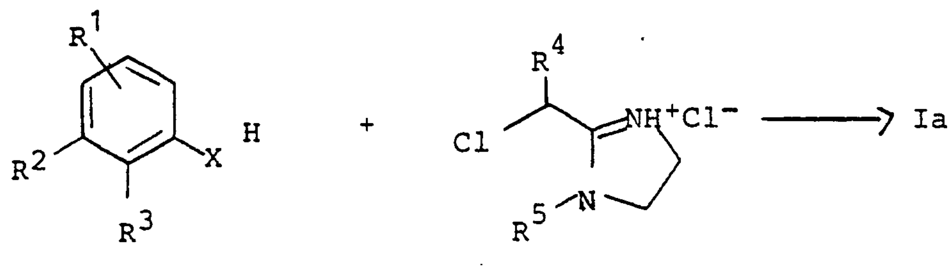

II III

oder

b) eine Arylverbindung der allgemeinen Formel V, wobei Y eine Carboxylgruppe oder ein reaktionsfähiges Derivat derselben, z.B. ein Imidat, Thioimidat, Ester, Orthoester, Thioamid, Imidohalgenid oder Amidin, ist und die Reste $R^1$ bis $R^5$ und X die oben festgelegte Bedeutung haben, mit einem Ethylendiamin der Formel IV oder einem Salz desselben umsetzt

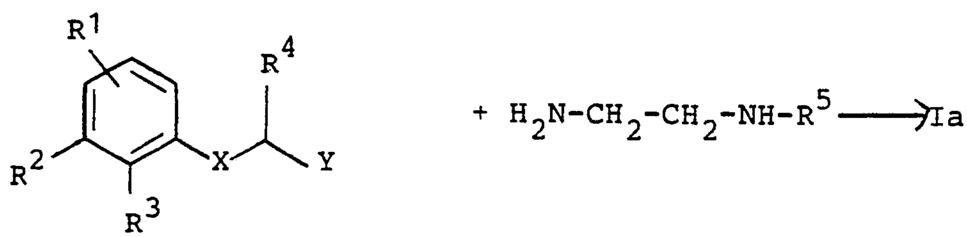

V VI

oder

c) ein Imidazolin der allgemeinen Formel VI mit einem Alkylhalogenid der allgemeinen Formel $R^5$-Z umsetzt, wobei die Reste $R^1$ bis $R^5$ und X die unter Formel Ia festgelegte Bedeutung haben und Z ein Halogenatom ist.

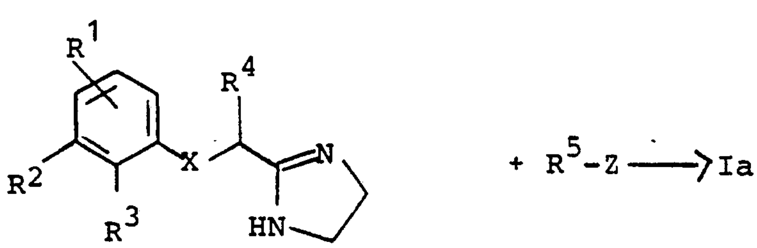

VI

4. Tierarzneimittel, dadurch gekennzeichnet, daß es eine Verbindung der Formel I nach Anspruch 1 enthält oder aus ihr besteht.

5. Verwendung von Verbindungen der Formel I nach Anspruch 1 zur wirtstiersystemischen Bekämpfung von Ektoparasiten.

Europäisches Patentamt

# EUROPÄISCHER RECHERCHENBERICHT

Nummer der Anmeldung

EP 89 12 3244

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | GB-A-1 180 862 (THE DOW CHEMICAL CO.) * Seite 4, Zeile 57 - Seite 60; Seite 6; Tabelle II, compound 5 * | 3 | C 07 D 233/22<br>A 01 N 43/50<br>C 07 D 233/20<br>C 07 D 233/24 |
| D,X | DE-A-2 750 902 (CIBA-GEIGY AG) * Seiten 1-3; Seiten 6-8; Seiten 11-14 * | 3-4 | |
| D,X | DE-A-2 818 367 (CIBA-GEIGY AG) * Seiten 1-9 * | 3-4 | |
| D,X | DE-A-2 756 638 (THE WELLCOME FOUNDATION LTD) * Seiten 1-2; Seiten 5-6; Seiten 8-9 * | 3-4 | |
| D,X | AT-A- 506 440 (TAKEDA CHEMICAL INDUSTRIES LTD) * Seiten 1Bn-2 * | 3 | |
| A | EP-A-0 049 797 (BAYER AG) | | |

RECHERCHIERTE SACHGEBIETE (Int. Cl.5)

C 07 D 233/00
A 01 N 43/00

Der vorliegende Recherchenbericht wurde für alle Patentansprüche erstellt

| Recherchenort | Abschlußdatum der Recherche | Prüfer |
|---|---|---|
| DEN HAAG | 23-03-1990 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)